# EUROPÄISCHE PATENTSCHRIFT

(11) **EP 3 038 742 B1**
(45) Veröffentlichungstag und Bekanntmachung des Hinweises auf die Patenterteilung: **07.06.2017**
(21) Anmeldenummer: 14755835.7
(22) Anmeldetag: 28.08.2014
(51) Int. Cl.: B01J 4/00, C07C 2/78

(54) **VORRICHTUNG UND VERFAHREN ZUM HERSTELLEN VON ACETYLEN UND SYNTHESEGAS**
DEVICE AND METHOD FOR PRODUCING ACETYLENES AND SYNTHESIS GAS
DISPOSITIF ET PROCÉDÉ DE PRODUCTION D'ACÉTYLÈNE ET DE GAZ DE SYNTHÈSE

(30) Priorität: 29.08.2013 EP 13182176
(43) Veröffentlichungstag der Anmeldung: 06.07.2016
(73) Patentinhaber: BASF SE, 67056 Ludwigshafen am Rhein (DE)
(72) Erfinder: KERN, Matthias, 67146 Deidesheim (DE); RUSS, Michael, 67354 Römerberg (DE); RENZE, Peter, 68163 Mannheim (DE); VICARI, Maximilian, 67117 Limburgerhof (DE)
(86) Internationale Anmeldenummer: PCT/EP2014/068232
(87) Internationale Veröffentlichungsnummer: WO 2015/028539

(56) Entgegenhaltungen:
- GB-A- 1 211 569
- US-A1- 2011 016 790
- US-A1- 2012 119 150

## Beschreibung

Die vorliegende Erfindung betrifft eine verbesserte Vorrichtung und ein verbessertes Verfahren zum Herstellen von Acetylen und Synthesegas durch partielle Oxidation von Kohlenwasserstoffen in einem Reaktor, bei welchen man dem Reaktor einen den Kohlenwasserstoff enthaltenden Strom sowie einen den Sauerstoff enthaltenden Strom zuleitet.

Hochtemperaturreaktionen zur partiellen Oxidation von Kohlenwasserstoffen werden üblicherweise in einem Reaktorsystem aus Mischeinheit, Brennerblock, Feuerraum und Quencheinrichtung durchgeführt. Als Beispiel für eine solche partielle Oxidation im Hochtemperaturbereich ist die Herstellung von Acetylen und Synthesegas durch partielle Oxidation von Kohlenwasserstoffen zu nennen. Diese wird beispielsweise in der DE 875198, DE 1051845, DE 1057094 und DE 4422815 beschrieben.

Hierin werden die für das BASF-Sachsse-Bartholomé-Acetylenverfahren üblicherweise eingesetzten Mischer/Brennerblock/Feuerraum/Quench-Kombinationen - im Folgenden, wenn auf die Kombination Bezug genommen wird, vereinfacht als Reaktor bezeichnet - erläutert.

Die Ausgangsstoffe, wie beispielsweise Erdgas und Sauerstoff werden dabei getrennt aufgeheizt, üblicherweise bis zu 600°C. In einer Mischzone werden die Reaktanden intensiv vermischt und nach Durchströmen eines Brennerblocks in einem Feuerraum zur exothermen Reaktion gebracht. Der Brennerblock besteht in diesen Fällen aus einer bestimmten Anzahl aus parallelen Kanälen, in denen die Strömungsgeschwindigkeit der zündfähigen Sauerstoff/Erdgas-Mischung höher ist als die Flammengeschwindigkeit (Reaktionsgeschwindigkeit, Umsetzungsgeschwindigkeit), um ein Durchschlagen der Flamme in den Mischraum zu verhindern. Der metallische Brennerblock wird gekühlt, um den thermischen Belastungen standzuhalten. Je nach Aufenthaltszeit im Mischraum entsteht die Gefahr der Vor- und Rückzündung aufgrund der begrenzten thermischen Stabilität der Mischungen. Hier wird der Begriff der Zündungsverzugszeit bzw. Induktionszeit gebraucht als diejenige Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden im Mischraum. Reaktanden wie Wasserstoff, Flüssiggas oder Leichtbenzin, deren Einsatz aufgrund von Ausbeute- und/oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch ihre zylinderförmige Geometrie des Feuerraums aus. Der Brennerblock weist vorzugsweise hexagonal angeordnete Durchführungsbohrungen auf. In einer Ausführungsform sind z. B. 127 Bohrungen mit jeweils einem Innendurchmesser von 27 mm hexagonal auf einem kreisförmigen Grundquerschnitt mit Durchmesser von ca. 500 mm angeordnet. In der Regel liegen die eingesetzten Bohrungs- oder Kanaldurchmesser bei etwa 19 mm bis 27 mm. Der anschließende Feuerraum, in dem die Flamme der acetylenbildenden partiellen Oxidationsreaktion stabilisiert wird, ist ebenfalls von zylindrischem Querschnitt, er ist wassergekühlt und entspricht im Erscheinungsbild dem eines kurzen Rohres (von z. B. 180 mm bis 533 mm Durchmesser und 380 bis 450 mm Länge). In Höhe der feuerraumseitigen Oberfläche des Brennerblocks wird sogenannter Hilfssauerstoff dem Reaktionsraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt. Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind außerhalb von dessen Umfang Quenchdüsen auf einem oder mehreren Quenchverteilerringen installiert, die das Quenchmedium, z. B. Wasser oder Öl, mit oder ohne Zuhilfenahme eines Zerstäubungsmediums zerstäuben und näherungsweise senkrecht zur Hauptströmungsrichtung der den Feuerraum verlassenden Reaktionsgase eindüsen. Dieser direkte Quench hat die Aufgabe, die reagierende Strömung extrem schnell auf ca. 100 °C (Wasserquench) und 200 °C (Ölquench) abzukühlen, so dass Folgereaktionen, d. h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Die im aktuellen Produktionsmaßstab eingesetzten Acetylenbrenner zeichnen sich durch eine zylinderförmige Geometrie des Feuerraums aus. Die Einsatzstoffe werden über einen Diffusor vorgemischt und unter Vermeidung von Rückvermischung dem Brennerblock über hexagonal angeordnete Durchführungsbohrungen zugeführt. Bei den bekannten Verfahren erfolgt die Vormischung der Einsatzstoffe im Mischdiffusor in einem relativ großen Volumen und unter hohen Vorwärmtemperaturen.

Bei dem beschriebenen technischen Verfahren entsteht neben Acetylen im Wesentlichen Wasserstoff, Kohlenmonoxid und Ruß. Die in der Flammenfront gebildeten Rußpartikel können als Keime an der feuerraumseitigen Oberfläche des Brennerblocks anhaften, worauf es zum Aufwachsen, Ablagern und Anbacken von Koksschichten kommt, wodurch das Verfahren in seiner Effektivität nachhaltig beeinträchtigt wird.

In den bestehenden Produktionsverfahren mit Öl- und Wasserquench werden diese Ablagerungen periodisch im Bereich der feuerraumseitigen Oberfläche des Brennerblocks mittels einer Stochereinrichtung mechanisch abgereinigt. Hierfür ist eine aufwändige Steuerung der Stochereinrichtung nötig (Ullmann's Encyclopedia of Industrial Chemistry, 5th Edition, Volume A1, pages 97-144) und zudem wird die jeweilige Einsatzzeit des Mechanismus' durch die thermische Belastung im Brennraum limitiert.

Es hat nicht an Versuchen gefehlt, den Nachteil des Anbackens von Koksschichten an der feuerraumseitigen Oberfläche des Brennerblocks zu vermeiden. So offenbart die Lehre der DE 2307300 das Injizieren einer gasförmigen Substanz in den Reaktor in einer Region zwischen maximaler Temperatur und Ort des Abschreckens. Hierbei soll es zu Reaktionen zwischen den zugegebenen Gasen und freien Radikalen kommen, wodurch die Koksbildung reduziert werden soll.

In der DE 3904330 A1 wird ein Verfahren zur Herstellung von Acetylenruß durch thermische Zersetzung von Acetylen beschrieben. Es wird hier bei diesem Verfahren, welches sich von dem Verfahren zur Herstellung von Acetylen deutlich unterscheidet (z. B. keine partielle Oxidation), erwähnt, dass man ggf. einen Inertgasstrom einleitet.

Die DE 1148229 beschreibt ein Verfahren zum Betreiben von Spaltkammern zur Behandlung von Kohlenwasserstoffen, wobei eine Spülung mit Wasserdampf vorgesehen ist und eine Kühlung der Wand zu einem Wasserschleier führen soll (Anspruch 1). Genauere Angaben über die Art der Ausführung der Spülung werden nicht gemacht. Es handelt sich bei dem vorgestellten Verfahren nicht um eine partielle Oxidation (POx), als Spülmedium wird flüssiges Wasser eingebracht und ein zusätzliches Beimischen eines Oxidationsmittels (z. B. Sauerstoff) zum Spülmedium ist nicht vorgesehen. Des Weiteren wird im axialen Verlauf der Spaltkammer nur an maximal einer Stelle ein Spülmedium injiziert.

In der DE 2007997 wird beschrieben, wie ein Ölfilm an der Innenwand der Reaktionskammer eine Verkokung vermeiden soll. Ein Ölfilm in einem Feuerraum neigt per se jedoch zur Verkokung. Daher kann ein kohlenwasserstoffhaltiges (Mineral-)Öl als Spülmedium bei der vorliegenden Herausforderung ausgeschlossen werden.

Die in den genannten Schriften offenbarten Verfahren zur Verhinderung bzw. Verringerung von unerwünschter Koksbildung befriedigen jedoch nicht bezüglich einer effektiven Anwendung beim Verfahren zur Herstellung von Acetylen. So beziehen sich einige der Schriften wie erläutert auf andere Reaktionen, wo gänzlich andere Bedingungen vorliegen und eine Übertragbarkeit nicht gegeben ist. So ist die bei dem erfindungsgemäßen Verfahren vorliegende partielle Oxidation in ihrer Charakteristik sehr anspruchsvoll: die Verweilzeiten spielen eine besonders große Rolle, der Abbruch der Reaktion muss sehr exakt erfolgen und die Zugabe von Fremdstoffen wie auch beispielsweise eines Spülgases oder Oxidators kann die Reaktion hinsichtlich ihres Ortes sowie ihrer Geschwindigkeit sehr schnell verschieben und damit zu einem Ausbeuteverlust führen.

Trotz der durch diese Vorrichtungen bewirkten Vorteile besteht nach wie vor ein Verbesserungspotenzial. Wie oben erwähnt, durchströmen die Reaktanden den Brennerblock durch Kanäle oder Bohrungen, um in einem Feuerraum zur exothermen Reaktion gebracht zu werden. Dieser Abschnitt des Brennerblocks wird auch als Lochbrennplatte bezeichnet. So wird zur Stabilisierung der sich im Rahmen der Acetylensynthese nach Sachsse-Bartholome ausbildenden Reaktionsfront der partiellen Oxidation im bestehenden Verfahren Hilfssauerstoff über einzelne Sauerstoffleitungen an vorbestimmten Positionen entlang der Lochbrennplatte des Reaktors in den Feuerraum bzw. Reaktionsraum eingebracht. Diese Leitungen werden radial von außen der Lochbrennplatte zugeführt. Hierdurch kommt es durch unterschiedliche Abstände der Austrittsöffnungen der Sauerstoffleitungen vom Umfang der Lochrennplatte zu unterschiedlichen Längen der Sauerstoffleitungen. Hierdurch variiert der Druckverlust der einzelnen Sauerstoffleitungen, die aus einem gemeinsamen Reservoir gespeist werden. Fällt der Unterschied der Längen der Sauerstoffleitungen zu groß aus, kommt es zu einer Fehlverteilung des Sauerstoffs über die Lochbrennplatte, wodurch sowohl die Flammenstabilität wie auch die gewünschte Ausbeute nicht mehr sichergestellt sind.

Es ist daher Aufgabe der vorliegenden Erfindung, eine Vorrichtung und ein Verfahren zum Herstellen von Acetylen und Synthesegas anzugeben, das die oben beschriebenen Nachteile zumindest weitgehend vermindert. Insbesondere ist die erfindungsgemäße Vorrichtung geeignet, eine Fehlverteilung des Sauerstoffs über die Lochbrennplatte zu vermeiden.

Ein Grundgedanke der vorliegenden Erfindung ist dabei, den Hilfssauerstoff nicht mehr über einzelne Leitungen zuzuführen, sondern mittels eines separaten Verteilerrings über einen Zwischenraum durch einzelne Bohrungen oder Kanäle in den Reaktionsraum einzubringen, wobei der Zwischenraum strömungsmechanisch derart ausgelegt ist, dass er eine Gleichverteilung auf die durch ihn versorgten Bohrungen gewährleistet.

Eine erfindungsgemäße Vorrichtung zum Herstellen von Acetylen und Synthesegas durch partielle Oxidation von Kohlenwasserstoffen mit Sauerstoff umfasst einen Reaktor gemäß Anspruch 1. Der Ringraum kann den Nebenraum umgeben. Beispielsweise umgibt der Ringraum den Nebenraum konzentrisch. Der Ringraum kann im Wesentlichen kreisförmig ausgebildet sein. Man beachte, dass sich eine derartige kreisförmige Ausbildung auf eine Schnittansicht senkrecht zu dem Verlauf einer Mittelachse oder Mittellinie des Ringraums bezieht. Im Allgemeinen ist der Ringraum zylindrisch ausgebildet und die Zylinderachse definiert die Mittelachse oder Mittellinie. Der Nebenraum ist von dem Ringraum durch eine Wand getrennt. Die Wand weist zum Verbinden der Bohrungen zum Zuführen eines Stroms aus Hilfssauerstoff mit dem Ringraum Öffnungen auf. Die Öffnungen können gleichmäßig entlang eines Umfangs des Brennerblocks in der Wand verteilt sein. Die Öffnungen können eine größere Querschnittsfläche als die Bohrungen zum Zuführen eines Stroms aus Hilfssauerstoff zu dem Feuerraum aufweisen. Das Verhältnis der Summe der Querschnittsflächen der Öffnungen zu der Querschnittsfläche des Ringraums kann von 0,05 bis 1 sein, beispielsweise 0,15. Der Ringraum ist mit einer Zuführung zum Zuführen von Hilfssauerstoff verbunden. Die Bohrungen zum Zuführen eines Stroms aus Hilfssauerstoff zu dem Feuerraum können in einem regelmäßigen oder unregelmäßigen Muster in dem Brennerblock angeordnet sein. Das Verhältnis der Summe der Querschnittsflächen der Bohrungen zum Zuführen eines Stroms aus Hilfssauerstoff zu dem Feuerraum zu der Querschnittsfläche des Brennerblocks kann von 0,0001 bis 0,1 sein, beispielsweise 0,015.

Ein erfindungsgemäßes Verfahren zum Herstellen von Acetylen und Synthesegas durch partielle Oxidation von Kohlenwasserstoffen mit Sauerstoff umfasst die folgenden Schritte. Die Ausgangsgase, die einen kohlenwasserstoffhaltigen Strom und einen sauerstoffhaltigen Strom umfassen, werden zunächst getrennt voneinander vorerhitzt, anschließend in einer Mischzone erhitzt, nach Durchströmen eines Brennerblocks in einem Feuerraum zur Reaktion gebracht und anschließend schnell abgekühlt. In dem Brennerblock sind dabei Bohrungen und ein Nebenraum ausgebildet. Der Nebenraum ist mit den Bohrungen und einem Ringraum verbunden, der den Nebenraum umgibt. Der Nebenraum ist so ausgebildet, dass ein Strom aus Hilfssauerstoff aus dem Ringraum durch die Bohrungen dem Feuerraum gleichmäßig verteilt zugeführt wird.

Der Ringraum kann den Nebenraum umgeben. Beispielsweise umgibt der Ringraum den Nebenraum konzentrisch. Der Ringraum kann mit dem Nebenraum über mehrere Öffnungen so verbunden sein, dass der Hilfssauerstoff aus dem Ringraum dem Nebenraum gleichmäßig verteilt zugeführt wird. Die Öffnungen können so angeordnet sein, dass der Hilfssauerstoff aus dem Ringraum in den Nebenraum in radialer Richtung zugeführt wird. Der Ringraum kann aus einer Zuführung zum Zuführen von Hilfssauerstoff gespeist werden.

Mischeinheit, Brennerblock, Feuerraum und Quencheinrichtung durchgeführt. Als Beispiel für eine solche partielle Oxidation im Hochtemperaturbereich ist die Herstellung von Acetylen und Synthesegas durch partielle Oxidation von Kohlenwasserstoffen zu nennen. Diese wird beispielsweise in der DE 875198, DE 1051845, DE 1057094 und DE 4422815 beschrieben.

Hierin werden die für das BASF-Sachsse-Bartholomé-Acetylenverfahren üblicherweise eingesetzten Mischer/Brennerblock/Feuerraum/Quench-Kombinationen - im Folgenden, wenn auf die Kombination Bezug genommen wird, vereinfacht als Reaktor bezeichnet - erläutert.

Unter einer Mischzone oder eine Mischeinheit eines Reaktors ist im Rahmen der vorliegenden Erfindung derjenige Bereich des Reaktors zu verstehen, in dem die getrennt aufgeheizten Ausgangsstoffe, wie beispielsweise Erdgas und Sauerstoff, intensiv vermischt werden.

Unter einem Brennerblock eines Reaktors ist im Rahmen der vorliegenden Erfindung derjenige Bereich des Reaktors zu verstehen, den die aufgeheizten und vermischten Ausgangsstoffe, durchströmen. Die vermischten Ausgangsstoffe strömen dabei in Kanälen oder Bohrungen.

Sofern nicht anders angegeben werden im Rahmen der vorliegenden Erfindung die Ausdrücke Kanal und Bohrung synonym verwendet. Unter einer Bohrung ist im Rahmen der vorliegenden Erfindung ein runder oder unrunder Durchbruch in einem Bauteil zu verstehen.

Unter einem Feuerraum eines Reaktors ist im Rahmen der vorliegenden Erfindung derjenige Bereich des Reaktors zu verstehen, in den die aufgeheizten und vermischten Ausgangsstoffe nach Durchströmen des Brennerblocks gelangen und zur exothermen Reaktion gebracht werden. Da im Rahmen der vorliegenden Erfindung als Ausgangstoffe Kohlenwasserstoff und Sauerstoff verwendet werden, bildet sich bei dieser exothermen Reaktion hauptsächlich Acetylen. Als Nebenprodukte entstehen Wasserstoff, Kohlenmonoxid und in geringem Umfang Ruß. Unter einer Querschnittsfläche ist im Rahmen der vorliegenden Erfindung der Flächeninhalt der bei einem Querschnitt freigelegten Fläche. Der Querschnitt verläuft dabei senkrecht zu einer Mittellinie desjenigen Bauteils, auf dessen Querschnittsfläche Bezug genommen wird. Beispielsweise ist die Querschnittsfläche einer Öffnung derjenige Flächeninhalt, der bei einem Querschnitt senkrecht zu einer gedachten Mittellinie durch den Mittelpunkt der Öffnung ermittelbar ist. Die Querschnittsfläche eines Ringraums ist derjenige Flächeninhalt, der bei einem Querschnitt senkrecht zu dessen Mittellinie, parallel zu der sich der Raum ringförmig erstreckt, ermittelbar ist. Die Querschnittsfläche eines Brennerblocks ist derjenige Flächeninhalt, der bei einem Querschnitt senkrecht zu dessen Mittellinie, die sich parallel zu einer Längserstreckungsrichtung des Brennerblocks erstreckt, ermittelbar ist. Die Längserstreckungsrichtung ist dabei eine Richtung parallel zu einer Zylinderachse des Brennerblocks, da dieser üblicherweise zylindrisch ausgebildet ist. Da der Brennerblock insbesondere rotationssymmetrisch ausgebildet ist, ist die Längserstreckungsrichtung parallel zu einer Rotationsachse des Brennerblocks bzw. des Brennermantels.

Unter einem regelmäßigen Muster ist im Rahmen der vorliegenden Erfindung ein Muster zu verstehen, das aus verschiedenen Elementen in einer vorbestimmten symmetrischen oder gleichmäßigen Ordnung besteht. Mit anderen Worten sind die Elemente in festen örtlichen Abständen zueinander wiederholt angeordnet.

Weitere optionale Einzelheiten und Merkmale der vorliegenden Erfindung ergeben sich aus der nachfolgenden Beschreibung bevorzugter Ausführungsbeispiele, welche schematisch in den Zeichnungen dargestellt sind.

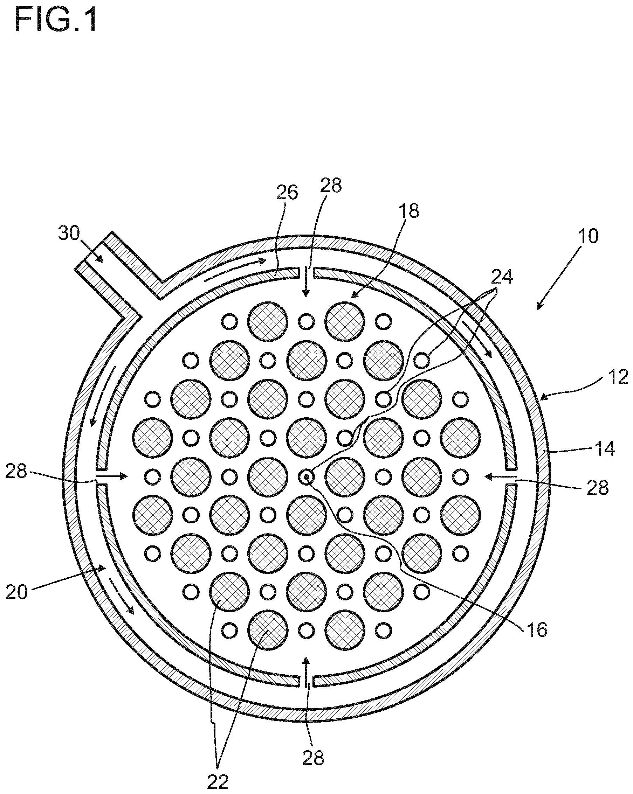

Es zeigt
- Figur 1: eine perspektivische Querschnittsansicht einer erfindungsgemäßen Vorrichtung zum Herstellen von Acetylen und Synthesegas durch partielle Oxidation von Kohlenwasserstoffen mit Sauerstoff.

### Ausführungsformen der Erfindung

Figur 1 zeigt eine Querschnittsansicht einer Vorrichtung 10 zum Herstellen von Acetylen und Synthesegas durch partielle Oxidation von Kohlenwasserstoffen mit Sauerstoff. Die Vorrichtung 10 umfasst einen Reaktor 12. Der Reaktor 12 weist einen Brennerblock 14 mit einem nicht näher gezeigten Feuerraum zur Acetylenherstellung auf. Der Brennerblock ist zylindrisch ausgebildet, so dass dieser eine Mittellinie 16 aufweist, die einer Zylinderachse der zylindrischen Form entspricht. Der Reaktor 12 weist weiter einen Nebenraum 18 auf. Der Nebenraum 18 ist in dem Brennerblock 14 ausgebildet. Der Reaktor 12 weist weiter einen Ringraum 20 auf. Der Ringraum 20 umgibt den Nebenraum 18. Beispielsweise umgibt der Ringraum 20 den Nebenraum 18 konzentrisch. Der Ringraum 20 ist bei dem in Figur 1 gezeigten Ausführungsbeispiel im Wesentlichen kreisförmig ausgebildet bezogen auf die Schnittdarstellung der Figur 1. Tatsächlich ist der Ringraum 20 bei einer dreidimensionalen Betrachtung zylindrisch ausgebildet.

Der Schnitt der Figur 1 verläuft senkrecht zu der Mittellinie 16 durch den Brennerblock 14. Der Feuerraum befindet sich bei der Schnittdarstellung der Figur 1 unterhalb der Zeichenebene, so dass dieser von dem Brennerblock 14 verdeckt ist und deshalb in der Figur 1 nicht zu sehen ist.

Der Brennerblock 14 weist Bohrungen 22 zum Zuführen eines Stroms einer Mischung aus Kohlenwasserstoffen und Sauerstoff zu dem Feuerraum auf. Die Bohrungen 22 zum Zuführen eines Stroms einer Mischung aus Kohlenwasserstoffen und Sauerstoff zu dem Feuerraum sind in einem regelmäßigen Muster in dem Brennerblock 14 angeordnet. Bei dem in Figur 1 gezeigten Ausführungsbeispiel sind die Bohrungen 22 zum Zuführen eines Stroms einer Mischung aus Kohlenwasserstoffen und Sauerstoff zu dem Feuerraum analog den Eckpunkten eines Quadrats um die Mittellinie 16 angeordnet, so dass die Mittellinie 16 durch den Mittelpunkt des Quadrats verläuft. Diese Anordnung wird beispielhaft an den bezüglich der Mittellinie 16 vier innersten Bohrungen 22 zum Zuführen eines Stroms einer Mischung aus Kohlenwasserstoffen und Sauerstoff zu dem Feuerraum näher erläutert. Die Mittelpunkte dieser vier innersten Bohrungen 22 bilden ein Quadrat, wobei die Mittellinie 16 durch den Mittelpunkt des Quadrats verläuft. Entsprechend ist der Abstand zwischen den Mittelpunkten von zwei unmittelbar benachbarten Bohrungen 22, d.h. Bohrungen 22 an den Enden einer Kante des Quadrats, um den Faktor √2 größer als der Abstand zwischen den Mittelpunkten von zwei Bohrungen 22, die sich mit dem Mittelpunkt des Quadrats zwischen sich gegenüberliegen, d.h. Bohrungen 22 an den Enden einer Diagonale des Quadrats. Die übrigen Bohrungen 22 sind analog angeordnet, so dass immer vier Bohrungen 22 mit ihren Mittelpunkten ein entsprechendes Quadrat bilden.

Der Brennerblock 14 weist weiter Bohrungen 24 zum Zuführen eines Stroms aus Hilfssauerstoff zu dem Feuerraum auf. Die Bohrungen 24 zum Zuführen eines Stroms aus Hilfssauerstoff zu dem Feuerraum sind mit dem Nebenraum 18 verbunden. Die Bohrungen 24 zum Zuführen eines Stroms aus Hilfssauerstoff zu dem Feuerraum sind ebenfalls in einem regelmäßigen Muster in dem Brennerblock 14 angeordnet. Alternativ können die Bohrungen 24 zum Zuführen eines Stroms aus Hilfssauerstoff zu dem Feuerraum in einem unregelmäßigen Muster angeordnet sein. Bei dem in Figur 1 gezeigten Ausführungsbeispiel sind die Bohrungen 24 zum Zuführen eines Stroms aus Hilfssauerstoff zu dem Feuerraum in Zwischenräumen zwischen den Bohrungen 22 zum Zuführen eines Stroms einer Mischung aus Kohlenwasserstoffen und Sauerstoff zu dem Feuerraum angeordnet. Die innerste Bohrung 24 bezüglich der Mittellinie 16 ist dabei so angeordnet, dass die Mittellinie 16 durch den Mittelpunkt der innersten Bohrung 24 verläuft. Die Bohrungen 24 sind analog den Eckpunkten eines Quadrats mit den Bohrungen 22 zum Zuführen eines Stroms einer Mischung aus Kohlenwasserstoffen und Sauerstoff zu dem Feuerraum zwischen sich angeordnet. Diese Anordnung wird beispielhaft vier Bohrungen 24 näher erläutert. Die Mittelpunkte dieser vier Bohrungen 24 bilden ein Quadrat, wobei der Mittelpunkt der Bohrung 22 mit dem Mittelpunkt des Quadrats zusammenfällt. Entsprechend ist der Abstand zwischen den Mittelpunkten von zwei unmittelbar benachbarten Bohrungen 24, d.h. Bohrungen 24 an den Enden einer Kante des Quadrats, um den Faktor √2 größer als der Abstand zwischen den Mittelpunkten von zwei Bohrungen 24, die sich mit dem Mittelpunkt der Bohrung 22 bzw. des Quadrats zwischen sich gegenüberliegen, d.h. Bohrungen 24 an den Enden einer Diagonale des Quadrats. Die übrigen Bohrungen 24 sind analog angeordnet, so dass immer vier Bohrungen 24 mit ihren Mittelpunkten ein entsprechendes Quadrat bilden.

Der Nebenraum 18 ist mit dem Ringraum 20 verbunden. Beispielsweise ist der Nebenraum 18 von dem Ringraum 20 durch eine Wand 26 getrennt. Die Wand 26 weist zum Verbinden der Bohrungen 24 zum Zuführen eines Stroms aus Hilfssauerstoff mit dem Ringraum 20 Öffnungen 28 auf. Beispielsweise sind die Öffnungen 28 entlang einer Umfangsrichtung des Brennerblocks 14 um die Mittellinie 16 in der Wand 26 gleichmäßig verteilt angeordnet. Bei dem in Figur 1 gezeigten Ausführungsbeispiel sind vier Öffnungen 28 vorgesehen, die gleichmäßig beabstandet entlang einer Umfangsrichtung des Brennerblocks 14 angeordnet sind, d. h. in einem Abstand von 90° bezogen auf die kreisförmige Ausbildung des Ringraums 20. Die Öffnungen 28 sind, wie in Figur 1, gezeigt so angeordnet, dass Hilfssauerstoff aus dem Ringraum 20 in den Nebenraum 18 in radialer Richtung bezüglich der Mittellinie 16 zuführbar ist. Die Öffnungen 28 weisen eine größere Querschnittsfläche als die Bohrungen 24 zum Zuführen eines Hilfssauerstoffs zu dem Feuerraum auf. Die Querschnittsfläche der Bohrungen 24 zum Zuführen eines Hilfssauerstoffs zu dem Feuerraum ist dabei der Flächeninhalt der Bohrungen 24 zum Zuführen eines Hilfssauerstoffs zu dem Feuerraum senkrecht zu der Mittellinie 16 bzw. parallel zu der Zeichenebene der Figur 1. Die Querschnittsfläche einer Öffnung 28 ist dabei der Flächeninhalt der Öffnung 28 parallel zu der Mittellinie 16 bzw. senkrecht zu der Zeicheneben der Figur 1 bzw. senkrecht zu der Strömungsrichtung eines durch die Öffnung 28 zugeführten Sauerstoffs. Das Verhältnis der Summe der Querschnittsflächen der Öffnungen 28 zu der Querschnittsfläche des Ringraums 20 kann von 0,05 bis 1, bevorzugt von 0,1 bis 0,4 und noch bevorzugter von 0,1 bis 0,2 sein, beispielsweise 0,15. Die Querschnittsfläche des Ringraums 20 ist dabei der Flächeninhalt des Ringraums 20 parallel zu der Mittellinie 16 bzw. senkrecht zu der Zeicheneben der Figur 1 bzw. senkrecht zu der Strömungsrichtung eines durch den Ringraum 20 strömenden Sauerstoffs. Das Verhältnis der Summe der Querschnittsflächen der Bohrungen 24 zum Zuführen eines Stroms aus Hilfssauerstoff zu dem Feuerraum 16 zu der Querschnittsfläche des Brennerblocks 14 kann von 0,0001 bis 0,1, bevorzugt von 0,05 bis 0,01 und noch bevorzugter von 0,02 bis 0,01 sein, beispielsweise 0,015. Die Querschnittsfläche des Brennerblocks 14 ist dabei der Flächeninhalt des Brennerblocks 14 senkrecht zu der Mittellinie 16 bzw. parallel zu der Zeichenebene der Figur 1.
Der Ringraum 20 ist mit einer Zuführung 30 zum Zuführen von Hilfssauerstoff verbunden. Die Zuführung 30 wird beispielsweise aus einem nicht näher gezeigten Reservoir gespeist, das als Sauerstoffleitung ausgeführt sein kann. Beispielsweise ist das Reservoir eine fest installierte Sauerstoffleitung aus einer Lufttrennungsanlage. Dadurch kann bei einer Zuführung von Hilfssauerstoff aus dem Ringraum 20 in den Nebenraum 18 nicht mehr Hilfssauerstoff aus dem Ringraum 20 in den Nebenraum 18 entweichen als aus dem Reservoir durch die Zuführung in den Ringraum 20 gelangt. Durch diese besondere geometrische Ausbildung werden somit Druckschwankungen in dem Ringraum 20 vermieden. Es wird explizit betont, dass auch mehr als eine Zuführung 30 mit dem Ringraum 20 verbunden sein kann.

Folglich wird der Hilfssauerstoff aus dem Ringraum 20 zunächst dem Nebenraum 18 zugeführt, wo sich der Hilfssauerstoff gleichmäßig verteilt, da dieser im Gegensatz zu den bekannten Vorrichtungen aus dem Stand der Technik nicht in Leitungen geführt wird. Da die Öffnungen 28 jeweils eine größere Querschnittsfläche als die Bohrungen 24 zum Zuführen eines Hilfssauerstoffs zu dem Feuerraum aufweisen, kann bei einer Zuführung von Hilfssauerstoff aus dem Nebenraum 18 durch die Bohrungen 24 zum Zuführen eines Hilfssauerstoffs zu dem Feuerraum in den Feuerraum nicht mehr Hilfssauerstoff aus dem Nebenraum 18 in den Feuerraum entweichen als aus dem Ringraum 20 durch die Öffnungen 28 in den Nebenraum 18 gelangt. Durch diese besondere geometrische Ausbildung werden somit Druckschwankungen in dem Nebenraum 18 vermieden. Der Nebenraum 18 ist somit strömungsmechanisch derart ausgelegt, dass er eine Gleichverteilung auf die durch den Nebenraum 18 versorgten Bohrungen 24 zum Zuführen eines Hilfssauerstoffs zu dem Feuerraum gewährleistet. Entsprechend werden Schwankungen des Drucks vermieden und die Flammenstabilität sowie die gewünschte Ausbeute werden verbessert.

Nachfolgend wird nun ein erfindungsgemäßes Verfahren zum Herstellen von Acetylen und Synthesegas durch partielle Oxidation von Kohlenwasserstoffen mit Sauerstoff beschrieben. Das grundlegende Verfahren basiert auf dem BASF-Sachsse-Bartholomé-Acetylenverfahren, weshalb auf diesbezügliche Einzelheiten nicht näher eingegangen wird, sondern auf die oben genannten Druckschriften verwiesen wird, deren Inhalt bezüglich des Verfahrens zum Herstellen von Acetylen und Synthesegas durch partielle Oxidation von Kohlenwasserstoffen mit Sauerstoff hierin durch Verweis eingeschlossen ist.

Die Ausgangsgase umfassen einen kohlenwasserstoffhaltigen Strom, wie beispielsweise einen Strom an Erdgas, und einen sauerstoffhaltigen Strom, wie beispielsweise einen Strom reinen Sauerstoffs. Diese Ausgangsgase werden zunächst getrennt voneinander vorerhitzt. Anschließend werden die vorerhitzten Ausgangsgase in einer nicht näher gezeigten Mischzone erhitzt. Die bereits erhitzten und gemischten Ausgangsgase durchströmen den Brennerblock 14 durch die Bohrungen 22 zum Zuführen eines Stroms einer Mischung aus Kohlenwasserstoffen und Sauerstoff zu dem Feuerraum und gelangen so in den Feuerraum. Außerdem wird der Ringraum 20 durch die Zuführung 30 mit Hilfssauerstoff aus dem Reservoir gespeist. Der Hilfssauerstoff strömt dann durch die Öffnungen 28 in der Wand 26 in den Nebenraum 18. Da mehrere Öffnungen 28 vorgesehen sind, die in der Wand 26 verteilt angeordnet sind, wird der Hilfssauerstoff dem Nebenraum 18 aus mehreren Richtungen zugeführt. Da der Hilfssauerstoff im Gegensatz zu den bekannten Vorrichtungen aus dem Stand der Technik nicht in Leitungen geführt wird, verteilt sich der Hilfssauerstoff gleichmäßig in dem Nebenraum 18. Dies wird auch durch die Öffnungen 28 unterstützt, die so angeordnet sind, dass der Hilfssauerstoff aus dem Ringraum 20 in den Nebenraum 18 in radialer Richtung bezüglich der Mittellinie 16 zugeführt wird. Der Hilfssauerstoff strömt dann aus dem Nebenraum 18 durch die Bohrungen 24 zum Zuführen eines Hilfssauerstoffs zu dem Feuerraum in den Feuerraum. Nach Durchströmen des Brennerblocks 14 werden die Ausgangsgase in dem Feuerraum zur Reaktion gebracht und anschließend durch eine Quencheinheit schnell abgekühlt. Des Weiteren wird der Hilfssauerstoff durch die Zuführung 30 in den Ringraum 20 zugeführt, so dass dieser bei einer Entnahme von Hilfssauerstoff aus dem Ringraum 20 in den Nebenraum 18 permanent gespeist wird.

Folglich wird also der Hilfssauerstoff aus dem Ringraum 20 zunächst dem Nebenraum 18 zugeführt, wo sich der Sauerstoff gleichmäßig verteilt. Durch die in dem Brennerblock 14 ausgebildeten Bohrungen 24 wird der Sauerstoff in den Feuerraum eingebracht. Der Nebenraum 18 ist dabei strömungsmechanisch derart ausgelegt, dass er eine Gleichverteilung auf die durch ihn versorgten Bohrungen 24 gewährleistet. Entsprechend werden Schwankungen des Drucks vermieden und die Flammenstabilität sowie die gewünschte Ausbeute werden verbessert.

### Bezugszeichenliste

- 10: Vorrichtung
- 12: Reaktor
- 14: Brennerblock
- 16: Mittellinie
- 18: Nebenraum
- 20: Ringraum
- 22: Bohrungen
- 24: Bohrungen
- 26: Wand
- 28: Öffnungen
- 30: Zuführung

## Patentansprüche

1. Vorrichtung (10) zum Herstellen von Acetylen und Synthesegas durch partielle Oxidation von Kohlenwasserstoffen mit Sauerstoff, umfassend einen Reaktor (12), wobei der Reaktor (12) einen Brennerblock (14) mit einem Feuerraum zur Acetylenherstellung, einen Nebenraum (18), der in dem Brennerblock (14) ausgebildet ist, und einen Ringraum (20), der den Nebenraum (18) umgibt, aufweist, wobei der Brennerblock (14) Bohrungen (22) zum Zuführen eines Stroms einer Mischung aus Kohlenwasserstoffen und Sauerstoff zu dem Feuerraum und Bohrungen (24) zum Zuführen eines Stroms aus Hilfssauerstoff zu dem Feuerraum aufweist, wobei die Bohrungen (24) zum Zuführen eines Stroms aus Hilfssauerstoff zu dem Feuerraum mit dem Nebenraum (18) verbunden sind, wobei der Nebenraum (18) mit dem Ringraum (20) verbunden ist, wobei der Nebenraum (18) von dem Ringraum (20) durch eine Wand (26) getrennt ist, wobei die Wand (26) zum Verbinden der Bohrungen (24) zum Zuführen eines Stroms aus Hilfssauerstoff mit dem Ringraum (20) Öffnungen (28) aufweist, wobei der Ringraum (20) mit mindestens einer Zuführung (30) zum Zuführen von Hilfssauerstoff verbunden ist.

2. Vorrichtung (10) nach dem vorhergehenden Anspruch, wobei der Ringraum (20) den Nebenraum (18) konzentrisch umgibt.

3. Vorrichtung (10) nach einem der vorhergehenden Ansprüche, wobei der Ringraum (20) im Wesentlichen kreisförmig ausgebildet ist.

4. Vorrichtung (10) nach dem vorhergehenden Anspruch, wobei die Öffnungen (28) entlang eines Umfangs des Brennerblocks (14) in der Wand (26) gleichmäßig verteilt sind.

5. Vorrichtung (10) nach einem der vorhergehenden Ansprüche, wobei die Öffnungen (28) eine größere Querschnittsfläche als die Bohrungen (24) zum Zuführen eines Stroms aus Hilfssauerstoff zu dem Feuerraum mit dem Ringraum (20) aufweisen.

6. Vorrichtung (10) nach einem der vorhergehenden Ansprüche, wobei das Verhältnis der Summe der Querschnittsflächen der Öffnungen (28) zu der Querschnittsfläche des Ringraums (20) von 0,05 bis 1 ist.

7. Vorrichtung (10) nach einem der vorhergehenden Ansprüche, wobei die Bohrungen (24) zum Zuführen eines Stroms aus Hilfssauerstoff zu dem Feuerraum in einem regelmäßigen oder unregelmäßigen Muster in dem Brennerblock (14) angeordnet sind.

8. Vorrichtung (10) nach einem der vorhergehenden Ansprüche, wobei das Verhältnis der Summe der Querschnittsflächen der Bohrungen (24) zum Zuführen eines Stroms aus Hilfssauerstoff zu dem Feuerraum zu der Querschnittsfläche des Brennerblocks (14) von 0,0001 bis 0,1 ist.

9. Verfahren zum Herstellen von Acetylen und Synthesegas durch partielle Oxidation von Kohlenwasserstoffen mit Sauerstoff, wobei die Ausgangsgase einen kohlenwasserstoffhaltigen Strom und einen sauerstoffhaltigen Strom umfassen, zunächst getrennt voneinander vorerhitzt, anschließend in einer Mischzone erhitzt werden, nach Durchströmen eines Brennerblocks (14) in einem Feuerraum zur Reaktion gebracht und anschließend schnell abgekühlt werden, wobei in dem Brennerblock (14) Bohrungen (24) und ein Nebenraum (18) ausgebildet sind, wobei der Nebenraum (18) mit den Bohrungen (24)und einem Ringraum (20) verbunden ist, der den Nebenraum (18) umgibt, wobei der Nebenraum (18) so ausgebildet ist, dass ein Strom aus Hilfssauerstoff aus dem Ringraum (20) durch die Bohrungen (24) dem Feuerraum gleichmäßig verteilt zugeführt wird.

10. Verfahren nach dem vorhergehenden Anspruch, wobei der Ringraum (20) den Nebenraum (18) konzentrisch umgibt.

11. Verfahren nach einem der beiden vorhergehenden Ansprüche, wobei der Ringraum (20) mit dem Nebenraum (18) über mehrere Öffnungen (28) so verbunden ist, dass der Hilfssauerstoff aus dem Ringraum (20) dem Nebenraum (18) gleichmäßig verteilt zugeführt wird.

12. Verfahren nach einem der drei vorhergehenden Ansprüche, wobei die Öffnungen (28) so angeordnet sind, dass der Hilfssauerstoff aus dem Ringraum (20) in den Nebenraum (18) in radialer Richtung zugeführt wird.

13. Verfahren nach einem der vier vorhergehenden Ansprüche, wobei der Ringraum (20) aus mindestens einer Zuführung (20) zum Zuführen von Hilfssauerstoff gespeist wird.

## Claims

1. An apparatus (10) for preparation of acetylene and synthesis gas by partial oxidation of hydrocarbons with oxygen, comprising a reactor (12), wherein the reactor (12) has a burner block (14) with a firing space for acetylene preparation, a secondary space (18) formed within the burner block (14), and an annular space (20) surrounding the secondary space (18), wherein the burner block (14) has holes (22) for supply of a stream of a mixture of hydrocarbons and oxygen to the firing space and holes (24) for supply of a stream of auxiliary oxygen to the firing space, wherein the holes (24) for supply of a stream of auxiliary oxygen to the firing space are connected to the secondary space (18), wherein the secondary space (18) is connected to the annular space (20), wherein the secondary space (18) is separated from the annular space (20) by a wall (26), said wall (26) having orifices (28) to connect the holes (24) for supply of a stream of auxiliary oxygen to the annular space (20), wherein the annular space (20) is connected to at least one feed (30) for supply of auxiliary oxygen.

2. The apparatus (10) according to the preceding claim, wherein the annular space (20) concentrically surrounds the secondary space (18).

3. The apparatus (10) according to either of the preceding claims, wherein the annular space (20) is essentially circular.

4. The apparatus (10) according to the preceding claim, wherein the orifices (28) are distributed homogeneously in the wall (26) along a circumference of the burner block (14).

5. The apparatus (10) according to any of the preceding claims, wherein the orifices (28) have a greater cross-sectional area than the holes (24) for supply of a stream of auxiliary oxygen to the firing space with the annular space (20).

6. The apparatus (10) according to any of the preceding claims, wherein the ratio of the sum total of the cross-sectional areas of the orifices (28) to the cross-sectional area of the annular space (20) is from 0.05 to 1.

7. The apparatus (10) according to any of the preceding claims, wherein the holes (24) for supply of a stream of auxiliary oxygen to the firing space are arranged in a regular or irregular pattern in the burner block (14).

8. The apparatus (10) according to any of the preceding claims, wherein the ratio of the sum total of the cross-sectional areas of the holes (24) for supply of a stream of auxiliary oxygen to the firing space to the cross-sectional area of the burner block (14) is from 0.0001 to 0.1.

9. A process for preparing acetylene and synthesis gas by partial oxidation of hydrocarbons with oxygen, wherein the starting gases comprise a hydrocarbonaceous stream and an oxygenous stream, are first preheated separately, then heated in a mixing zone, flow through a burner block (14) and then are reacted in a firing space and subsequently cooled rapidly, wherein holes (24) and a secondary space (18) are formed in the burner block (14), wherein the secondary space (18) is connected to the holes (24) and an annular space (20) surrounding the secondary space (18), wherein the secondary space (18) is formed such that a stream of auxiliary oxygen from the annular space (20) is fed to the firing space in homogeneous distribution through the holes (24).

10. The process according to the preceding claim, wherein the annular space (20) concentrically surrounds the secondary space (18).

11. The process according to either of the two preceding claims, wherein the annular space (20) is connected to the secondary space (18) via a plurality of orifices (28) such that the auxiliary oxygen from the annular space (20) is fed to the secondary space (18) in homogeneous distribution.

12. The process according to any of the three preceding claims, wherein the orifices (28) are arranged such that the auxiliary oxygen from the annular space (20) is fed into the secondary space (18) in radial direction.

13. The process according to any of the four preceding claims, wherein the annular space (20) is fed from at least one feed (20) for supply of auxiliary oxygen.

## Revendications

1. Dispositif (10) pour la fabrication d'acétylène et de gaz de synthèse par oxydation partielle d'hydrocarbures avec de l'oxygène, comprenant un réacteur (12), le réacteur (12) comprenant un bloc brûleur (14) qui comprend une chambre de combustion pour la fabrication d'acétylène, une chambre secondaire (18), qui est configurée dans le bloc brûleur (14), et une chambre annulaire (20), qui entoure la chambre secondaire (18), le bloc brûleur (14) comprenant des alésages (22) pour l'introduction d'un courant d'un mélange d'hydrocarbures et d'oxygène dans la chambre de combustion et des alésages (24) pour l'introduction d'un courant d'oxygène auxiliaire dans la chambre de combustion, les alésages (24) pour l'introduction d'un courant d'oxygène auxiliaire dans la chambre de combustion étant raccordés avec la chambre secondaire (18), la chambre secondaire (18) étant raccordée avec la chambre annulaire (20), la chambre secondaire (18) étant séparée de la chambre annulaire (20) par une paroi (26), la paroi (26) comprenant des ouvertures (28) pour le raccord des alésages (24) pour l'introduction d'un courant d'oxygène auxiliaire avec la chambre annulaire (20), la chambre annulaire (20) étant raccordée avec au moins une conduite d'alimentation (30) pour l'introduction d'oxygène auxiliaire.

2. Dispositif (10) selon la revendication précédente, dans lequel la chambre annulaire (20) entoure concentriquement la chambre secondaire (18).

3. Dispositif (10) selon l'une quelconque des revendications précédentes, dans lequel la chambre annulaire (20) est configurée sous forme essentiellement circulaire.

4. Dispositif (10) selon la revendication précédente, dans lequel les ouvertures (28) sont réparties uniformément le long d'une périphérie du bloc brûleur (14) dans la paroi (26).

5. Dispositif (10) selon l'une quelconque des revendications précédentes, dans lequel les ouvertures (28) présentent une surface de section transversale plus grande que les alésages (24) pour l'introduction d'un courant d'oxygène auxiliaire dans la chambre de combustion avec la chambre annulaire (20).

6. Dispositif (10) selon l'une quelconque des revendications précédentes, dans lequel le rapport entre la somme des surfaces de section transversale des ouvertures (28) et la surface de section transversale de la chambre annulaire (20) est de 0,05 à 1.

7. Dispositif (10) selon l'une quelconque des revendications précédentes, dans lequel les alésages (24) pour l'introduction d'un courant d'oxygène auxiliaire dans la chambre de combustion sont agencés selon un motif régulier ou irrégulier dans le bloc brûleur (14).

8. Dispositif (10) selon l'une quelconque des revendications précédentes, dans lequel le rapport entre la somme des surfaces de section transversale des alésages (24) pour l'introduction d'un courant d'oxygène auxiliaire dans la chambre de combustion et la surface de section transversale du bloc brûleur (14) est de 0,0001 à 0,1.

9. Procédé de fabrication d'acétylène et de gaz de synthèse par oxydation partielle d'hydrocarbures avec de l'oxygène, dans lequel les gaz de départ comprennent un courant contenant des hydrocarbures et un courant contenant de l'oxygène, sont tout d'abord préchauffés séparément l'un de l'autre, puis chauffés dans une zone de mélange, mis en réaction dans une chambre de combustion après avoir traversé un bloc brûleur (14), puis refroidis rapidement, des alésages (24) et une chambre secondaire (18) étant configurés dans le bloc brûleur (14), la chambre secondaire (18) étant raccordée avec les alésages (24) et une chambre annulaire (20), qui entoure la chambre secondaire (18), la chambre secondaire (18) étant configurée de sorte qu'un courant d'oxygène auxiliaire issu de la chambre annulaire (20) soit introduit dans la chambre de combustion sous forme distribuée uniformément par les alésages (24).

10. Procédé selon la revendication précédente, dans lequel la chambre annulaire (20) entoure concentriquement la chambre secondaire (18).

11. Procédé selon l'une quelconque des deux revendications précédentes, dans lequel la chambre annulaire (20) est raccordée avec la chambre secondaire (18) par plusieurs ouvertures (28) de sorte que l'oxygène auxiliaire issu de la chambre annulaire (20) soit introduit sous forme distribuée uniformément dans la chambre secondaire (18).

12. Procédé selon l'une quelconque des trois revendications précédentes, dans lequel les ouvertures (28) sont agencées de sorte que l'oxygène auxiliaire issu de la chambre annulaire (20) soit introduit en direction radiale dans la chambre secondaire (18).

13. Procédé selon l'une quelconque des quatre revendications précédentes, dans lequel la chambre annulaire (20) est alimentée par au moins une conduite d'alimentation (20) pour l'alimentation d'oxygène auxiliaire.
